(19) **Europäisches Patentamt**
**European Patent Office**
**Office européen des brevets**

(11) **EP 4 043 163 B1**

(12) **EUROPEAN PATENT SPECIFICATION**

(45) Date of publication and mention
of the grant of the patent:
**10.07.2024 Bulletin 2024/28**

(21) Application number: **20877761.5**

(22) Date of filing: **18.09.2020**

(51) International Patent Classification (IPC):
***B25J 13/08*** (2006.01)       ***A61B 34/37*** (2016.01)

(52) Cooperative Patent Classification (CPC):
**A61B 34/30; A61B 34/37;** A61B 2034/305;
A61B 2090/064; A61B 2090/066

(86) International application number:
**PCT/JP2020/035626**

(87) International publication number:
**WO 2021/075213 (22.04.2021 Gazette 2021/16)**

(54) **SURGICAL ROBOT SYSTEM, EXTERNAL FORCE ESTIMATION DEVICE, AND PROGRAM**

CHIRURGISCHES ROBOTERSYSTEM, EXTERNE KRAFTMESSVORRICHTUNG UND
PROGRAMM

SYSTÈME DE ROBOT CHIRURGICAL, DISPOSITIF D'ESTIMATION DE FORCE EXTERNE ET
PROGRAMME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.10.2019 JP 2019190341**

(43) Date of publication of application:
**17.08.2022 Bulletin 2022/33**

(73) Proprietor: **RIVERFIELD Inc.**
**1070052 Tokyo (JP)**

(72) Inventors:
• **HARAGUCHI, Daisuke**
**Tokyo 160-0017 (JP)**
• **TADANO, Kotaro**
**Tokyo 160-0017 (JP)**
• **AIZAWA, Koki**
**Tokyo 160-0017 (JP)**

(74) Representative: **adares Patent- und**
**Rechtsanwälte**
**Reininger & Partner GmbB**
**Tauentzienstraße 7 b/c**
**10789 Berlin (DE)**

(56) References cited:
**EP-A1- 1 915 963        EP-A1- 3 205 459**
**WO-A1-2015/079775    WO-A1-2015/079775**

WO-A1-2016/056339    WO-A1-2018/159336
WO-A1-2019/056871    JP-A- 2010 507 792
US-A1- 2019 060 019

• **BAUZANO E ET AL: "Active wrists endoscope
navigation in robotized laparoscopic surgery",
MECHATRONICS, 2009. ICM 2009. IEEE
INTERNATIONAL CONFERENCE ON, IEEE,
PISCATAWAY, NJ, USA, 14 April 2009
(2009-04-14), pages 1 - 6, XP031457256, ISBN:
978-1-4244-4194-5**
• **MAGO JESUS ET AL: "Safe teleoperation of a
laparoscope holder with dynamic precision but
low stiffness", 2019 INTERNATIONAL
CONFERENCE ON ROBOTICS AND
AUTOMATION (ICRA), IEEE, 20 May 2019
(2019-05-20), pages 2693 - 2699, XP033594017,
DOI: 10.1109/ICRA.2019.8794076**
• **KIM SUYONG ET AL: "A 3-DOF sensor to estimate
the force applied to the tip of a surgical
instrument", 2017 18TH INTERNATIONAL
CONFERENCE ON ADVANCED ROBOTICS
(ICAR), IEEE, 10 July 2017 (2017-07-10), pages
143 - 148, XP033147082, DOI:
10.1109/ICAR.2017.8023509**

EP 4 043 163 B1

- **KIM SUYONG ET AL: "A method to estimate the axial force applied to a surgical instrument tip considering the effect of the gravity", 2017 11TH ASIAN CONTROL CONFERENCE (ASCC), IEEE, 17 December 2017 (2017-12-17), pages 1246 - 1251, XP033314529, DOI: 10.1109/ASCC.2017.8287349**
- **MAGO JESUS, ARICO MARIO, SILVA JIMMY DA, MOREL GUILLAUME: "Safe teleoperation of a laparoscope holder with dynamic precision but low stiffness", 2019 INTERNATIONAL CONFERENCE ON ROBOTICS AND AUTOMATION (ICRA, 24 May 2019 (2019-05-24), pages 2693 - 2699, XP033594017, ISSN: 1050-4729**

**Description**

TECHNICAL FIELD

The present disclosure relates to a surgical robot system, an external force estimation device, and a program.

BACKGROUND ART

In recent years, endoscopic surgery using a surgical robot system is becoming widespread. Used in the endoscopic surgery is a laparoscope, an endoscope, forceps, and so on (hereinafter also referred to as "surgical instruments") each attached to an arm device of the surgical robot system (see, for example, Patent Document 1, and Documents 5 to 9).

Patent Document 2 describes a minimally invasive medical system comprising a manipulator having an effector unit equipped with a 6-DOF force/torque sensor and configured for holding a minimally invasive instrument having a first end mounted to the effector unit and a second end located beyond an external fulcrum that limits the instrument in motion. This system comprises a programmable computing device which is programmed to determine a position of the instrument relative to the fulcrum; process measurements made by means of the 6-DOF force/torque sensor of a force and a torque exerted onto the effector unit by the first end of the instrument; and to calculate by means of the principle of superposition an estimate of a force exerted onto the second end of the instrument based on the determined position, the measured force and the measured torque.

Patent document 3 describes an information processing device comprising an acting force calculation unit that calculates, on a basis of a first detected value by a first force sensor provided on one side of a rod-shaped member, at least one of acting forces on a first point of action and a second point of action that differ from each other on another side of the rod-shaped member.

Patent document 4 discloses a surgical robot comprising an external force calculation means that calculates external force such that the resultant force of forceps is zero; an external force action point calculation means that calculates an external force action point such that the total moment of the forceps is zero; a contact determination means that determines whether or not an object has come in contact with between the tip and the base of the forceps; and a warning means that, if a determination is made by the contact determination means that there has been contact, warns of forceps contact.

PRIOR ART DOCUMENTS

PATENT DOCUMENTS

Patent Document 1: Japanese Translation of PCT International Application Publication No. 2009-522016
Patent Document 2: EP 1 915 963 A1
Patent Document 3: EP 3 205 459 A1
Patent Document 4: WO 2015/079775 A1
Document 5: BAUZANO E. ET AL: "Active wrists endoscope navigation in robotized laparoscopic surgery", MECHATRONICS, 2009. ICM 2009. IEEE INTERNATIONAL CONFERENCE ON, IEEE, PISCATAWAY, NJ, USA, 14. April 2009, pages 1-6, ISBN: 978-1-4244-4194-5
Document 6: MAGO JESUS ET AL: "Safe teleoperation of a laparoscope holder with dynamic precision but low stiffness", 2019 INTERNATIONAL CONFERENCE ON ROBOTICS AND AUTOMATION (ICRA), IEEE, 20. May 2019, pages 2693-2699
Patent Document 7: WO 2019/056871 A1
Document 8: KIM SUYONG ET AL: "A 3-DOF sensor to estimate the force applied to the tip of a surgical instrument", 18TH INTERNATIONAL CONFERENCE ON ADVANCED ROBOTICS (ICAR), IEEE, 10. July 2017, pages 143-148
Document 9: KIM SUYONG ET AL: "A method to estimate the axial force applied to a surgical instrument tip considering the effect of the gravity", 11TH ASIAN CONTROL CONFERENCE (ASCC), IEEE, 17. December 2017, pages 1246-1251.

SUMMARY OF THE INVENTION

PROBLEMS TO BE SOLVED BY THE INVENTION

A trocar (or a port) through which the surgical instrument is inserted is placed in a subject (e.g., through an abdominal wall) to be subjected to the endoscopic surgery. In surgical robot systems as disclosed in Patent Document 1, it is desirable to be able to reduce a load, specifically a force, applied to the subject by the surgical instrument during the surgery.

Examples of the force applied may include a force applied to the abdominal wall of the subject via the trocar, and a force applied directly to the subject by a part of the surgical instrument not at a position corresponding to the trocar (e.g., a distal end part or a shaft of the surgical instrument).

Known as methods for detecting a force applied by the distal end part of the surgical instrument, in other words a force applied to the distal end part of the surgical instrument, are a method of measuring by a sensor arranged in the neighborhood of the distal end part, and a method of estimating an external force applied to a joint of the distal end part based on a driving force.

However, the method of measuring by a sensor arranged in the neighborhood of the distal end part makes it necessary to attach the sensor for measurement directly to the neighborhood of the distal end part of the surgical instrument. Such an arrangement position of the sensor causes a problem of difficulty in reducing the size of the distal end part of the surgical instrument and a problem of difficulty in sterilizing and washing the surgical instrument. This results in further problems, such as increased costs for manufacture and use.

The method of estimating an external force applied to the joint has problems such as variability of accuracy in estimating the external force under the influence of, for example, friction within a mechanism of the surgical instrument and so on, and presence of an undetectable force direction depending on arrangement of degrees of freedom.

Moreover, in the method of measuring by a sensor arranged in the neighborhood of the distal end part or in the method of estimating the external force applied to the joint, a force applied to the shaft in a middle area cannot be measured or estimated, respectively. Thus, an estimated value of the external force could result in being smaller than an actual value of the external force.

In one aspect of the present disclosure, it is desirable to provide a surgical robot system, an external force estimation device, and a program that make it easier to try to reduce the size of a distal end of the surgical instrument and to improve accuracy in measurement of an external force acting on the surgical instrument, and that can make it easier to try to enhance the safety.

MEANS FOR SOLVING THE PROBLEMS

The problems are solved by an external force estimation device according to claim 1, a surgical robot system according to claim 2, and a program according to claim 5.

The surgical robot system according to a first aspect as one aspect of the present disclosure comprises: a surgical instrument used in surgery on a subject, the surgical instrument including a shaft formed in a long shape, the shaft being inserted through a trocar placed in the subject; an arm portion configured to grasp the surgical instrument in a relatively movable manner with respect to the subject; a detector arranged at a position not where the shaft is present but where the arm is present and configured to detect values of forces and torques acting on the surgical instrument, the detector detecting the values of: a length-direction force acting in a length direction of the shaft, a length-direction torque acting about the length direction, a first intersecting-direction force acting in a first intersecting direction that intersects with the length direction, a first intersecting-direction torque acting about the first intersecting direction, a second intersecting-direction force acting in a second intersecting direction that intersects with the length direction and with the first intersecting direction, and a second intersecting-direction torque acting about the second intersecting direction; and a calculator configured to calculate a value of a first external force that the surgical instrument receives from the trocar and a value of a second external force that the surgical instrument receives from a portion other than the trocar based on the values of the length-direction force, the first intersecting-direction force, the second intersecting-direction force, the length-direction torque, the first intersecting-direction torque, and the second intersecting-direction torque, and on a value of a resistance force acting between the trocar and the shaft.

The external force estimation device according to a second aspect of the present disclosure comprises: an inputter configured to receive input of values of forces and torques detected by a detector, the detector being arranged at a position not where a shaft of a surgical instrument is present but where the arm is present, the shaft being formed in a long shape and inserted through a trocar placed in a subject, the values being values of: a length-direction force acting in a length direction of the shaft, a length-direction torque acting about the length direction, a first intersecting-direction force acting in a first intersecting direction that intersects with the length direction, a first intersecting-direction torque acting about the first intersecting direction, a second intersecting-direction force acting in a second intersecting direction that intersects with the length direction and with the first intersecting direction, and a second intersecting-direction torque acting about the second intersecting direction; and a calculator configured to calculate a value of a first external force that the surgical instrument receives from the trocar and a value of a second external force that the surgical instrument receives from a portion other than the trocar based on the values of the length-direction force, the first intersecting-direction force, the second intersecting-direction force, the length-direction torque, the first intersecting-direction torque, and the second intersecting-direction torque, and on a value of a resistance force acting between the trocar and the shaft.

The program according to a third aspect of the present disclosure is configured to cause a computer to perform: an input function of inputting values of forces and torques detected by a detector, the detector being arranged at a position not where a shaft of a surgical instrument is present but where the arm is present, the shaft being formed in a long shape and inserted through a trocar placed in a subject, the values being values of: a length-direction force acting in a length direction of the shaft, a length-direction torque acting about the length direction, a first intersecting-direction force acting in a first intersecting direction that intersects with the length direction, a first intersecting-direction torque acting about the first intersecting direction, a second intersecting-direction force acting in a second intersecting direction that intersects with the length direction and with the first intersecting direction, and a second intersecting-direction torque acting about the second intersecting direction; and a calculation function of calculating a value of a first external force that the surgical instrument receives from the trocar and a value of a second external force that the surgical instrument receives from a portion other than the trocar based on the values of the length-direction force, the first intersecting-direction force, the second intersecting-direction force, the length-direction torque, the first intersecting-direction torque, and the second intersecting-direction torque, and on a value of a resistance force acting between the trocar and the shaft.

The surgical robot system according to the first aspect, the external force estimation device according to the second aspect, and the program according to the third aspect of the present disclosure make it possible to calculate the value of the first external force that the surgical instrument receives from the trocar and the value of the second external force that the surgical instrument receives from a portion other than the trocar based on values of various external forces and torques detected by the detector and on the value of the resistance force acting between the trocar and the shaft.

Further, since the detector for detecting the values of the various external forces and torques is arranged at a position not where the shaft is present but where the arm is present, an attempt to reduce the size of the surgical instrument is facilitated, as compared with a case where a sensor is arranged at a distal end of the surgical instrument.

In the above-described first aspect of the disclosure, it is preferred that a drive portion configured to drive a distal end part of the shaft of the surgical instrument be provided, that the arm portion comprise a holder configured to hold the surgical instrument, and that the detector be arranged between the drive portion and the holder.

Such arrangement of the detector between the drive portion and the holder makes it less likely for the detector to detect a force and a torque caused by operation of the drive portion, as compared with a case where, for example, the detector is arranged closer to the distal end part than the drive portion is. In other words, improvement in accuracy of detection by the detector is facilitated.

In the above-described first aspect of the disclosure, it is preferred that a distal end external force detector configured to detect a value of a distal end external force, which is an external force that the distal end part receives be provided, and that the calculator calculate a value of a shaft external force, which is an external force in the shaft received from a portion other than the distal end part and the trocar, based on the value of the second external force and the value of the distal end external force.

Such provision of the distal end external force detector enables the calculator to calculate the value of the shaft external force, which is an external force in the shaft received from a portion other than the distal end part and the trocar. Examples of the shaft external force may include a force applied by contact of a midsection of the shaft with an organ or the like of the subject, caused by relative movement of the surgical instrument with respect to the subject.

A storage section in which values of resistance forces each acting between the trocar of a different type and the shaft are stored in association with types of the trocar and a selector used to select the type of the trocar be provided, and that the calculator acquire, from the storage section, the value of the resistance force for the type of the trocar selected by the selector and calculate the first external force and the second external force using the value of the resistance force acquired.

Such provision of the storage section and the selector makes it easier to inhibit deterioration in accuracy of the calculated values of the first external force and the second external force, even when the trocar to be used is changed to the trocar different in the resistance force acting between the trocar and the shaft.

EFFECTS OF THE INVENTION

In the surgical robot system according to the first aspect, the external force estimation device according to the second aspect, and the program according to the third aspect of the present disclosure, the value of the first external force that the surgical instrument receives from the trocar and the value of the second external force that the surgical instrument receives from a portion other than the trocar are calculated based on the values of the various external forces and torques detected by the detector and on the value of the resistance force acting between the trocar and the shaft. This produces effects of making it easier to try to reduce the size of the distal end of the surgical instrument and to improve accuracy in measurement of an external force acting on the surgical instrument, and of making it easier to try to enhance the safety.

BRIEF DESCRIPTION OF THE DRAWINGS

FIG. 1 is a schematic diagram explaining a configuration of a surgical robot system according to a first embodiment of the present disclosure.

FIG. 2 is a diagram explaining a coordinate system, forces, and torques in the surgical robot system in FIG. 1.

FIG. 3 is a block diagram explaining a configuration of a controller in FIG. 1.

FIG. 4 is a schematic diagram explaining a configuration of a surgical robot system according to a second embodiment of the present disclosure.

FIG. 5 is a block diagram explaining a configuration of a controller in FIG. 4.

FIG. 6 is a schematic diagram explaining a configuration of a surgical robot system according to an embodiment of the present invention.

FIG. 7 is a block diagram explaining a configuration of a controller in FIG. 6.

EXPLANATION OF REFERENCE NUMERALS

1, 101, 201...surgical robot system, 10...surgical instrument, 11...shaft, 30, 130...arm device (arm portion), 45...holder, 46...detector, 48...drive portion, 50, 150, 250...controller (external force estimation device), 51, 151...inputter, 53, 153, 253...calculator, 70...patient (subject), 75...trocar, 148...distal end external force detector, 251...selector, Fp...first external force, Fr...second external force, $^5Fpx$...resistance force

MODE FOR CARRYING OUT THE INVENTION

[First Embodiment]

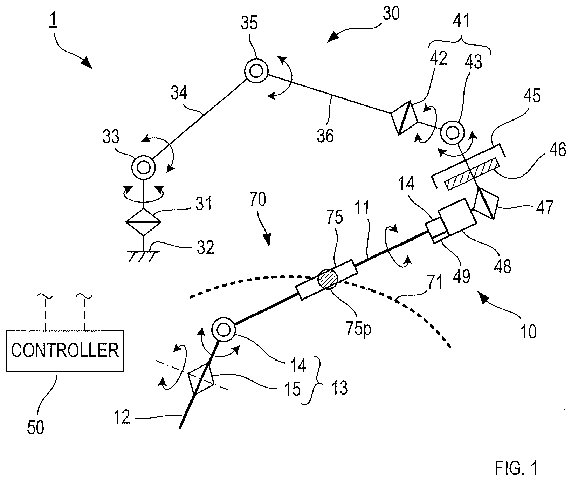

A surgical robot system 1 according to a first embodiment of the present disclosure will be described with reference to FIG. 1 to FIG. 3. In the present embodiment, the surgical robot system 1 is a surgery assisting robot or the like used in endoscopic surgery. As shown in FIG. 1, the surgical robot system 1 comprises a surgical instrument 10, an arm device 30, and a controller 50. The arm device 30 and the controller 50 each correspond to one example of an arm portion and an external force estimation device, respectively.

The surgical instrument 10 is an instrument of every kind, such as an endoscope or forceps, used in endoscopic surgery. In the present embodiment, an explanation will be given of an example in which the surgical instrument 10 is forceps. As shown in FIG. 1, the surgical instrument 10 comprises a shaft 11 and a main body 14.

The shaft 11 is a member extending in a tubular shape or a rod shape, and is inserted through a trocar 75 to thereby be inserted inside an abdominal wall 71 of a patient 70. The shaft 11 is provided with a distal end part 12, which is forceps, and a bend portion 13.

The trocar is hereinafter also referred to as a port. The patient 70 corresponds to one example of a subject.

The distal end part 12 is a part at a distal end, which is an end of the shaft 11 to be inserted into the patient 70. In the present embodiment, the distal end part 12 is provided with an element for gripping an object.

The bend portion 13 has a shape of a cylinder or a column, with a first end thereof arranged on the distal end part 12 and a second end thereof arranged on the shaft 11. The bend portion 13 has a configuration capable of bending laterally with respect to a direction in which the shaft 11 extends. FIG. 1 shows an example in which the bend portion 13 is configured with two rotating portions 14 and 15, rotation axes of which intersect with each other, and more preferably, intersect perpendicularly with each other. The configuration of the bend portion 13 is not particularly limited to this configuration.

The main body 14 is a part, in the surgical instrument 10, to be attached to and detached from the arm device 30. The main body 14 has a configuration through which a driving force to drive the distal end part 12 and the bend portion 13 transmitted from the arm device 30 can be transmitted. A specific configuration of the main body 14 is not limited to a particular one.

The arm device 30 supports the surgical instrument 10 such that a position and an attitude thereof are changeable. The arm device 30 is controlled so that the surgical instrument 10 passes through a pivot position 75p, which is a specified relative position with respect to the arm device 30, even when the position and/or the attitude of the surgical instrument 10 are/is changed. The pivot position 75p is hereinafter also referred to as an immovable position.

In the present embodiment, the arm device 30 is configured such that all of a rotating portion 31, a first arm rotating portion 33, a second arm rotating portion 35, and so on, which will be described below, are controlled so that the surgical instrument 10 passes through the pivot position 75p. However, it suffices if the surgical instrument 10 at least passes through the pivot position 75p, and thus, the arm device 30 does not necessarily have to be configured such that all of the rotating portion 31, the first arm rotating portion 33, the second arm rotating portion 35, and so on are controlled actively.

The pivot position 75p may be predetermined or may be yet to be determined. In other words, the pivot position 75p may be a position provided in advance, or may be a position obtained based on a value measured by any sensor. The pivot position 75p may be a position estimated based on behavior of the arm device 30.

The pivot position 75p generally coincides with a positon of an insertion hole in the abdominal wall 71 of the patient 70 subjected to endoscopic surgery. The trocar 75 through which the shaft 11 of the surgical instrument 10 is inserted is placed in the insertion hole.

The trocar 75 of the present embodiment comes into contact with the shaft 11 of the surgical instrument 10 inserted, and is not coupled and fixed to the arm device 30. That is, the trocar 75 is not held by the arm device 30.

In other words, the trocar 75 is used together with the arm device 30 having no need for pivot positioning, that is, the arm device 30 having no need for alignment of an immovable point. Since the trocar 75 and the arm device 30 are not coupled and fixed to each other, improvement in accuracy of calculation of a first external force Fp and a second external force Fr is facilitated as compared with a case where they are coupled and fixed to each other.

As shown in FIG. 1, the arm device 30 comprises the rotating portion 31, the first arm rotating portion 33, a first arm 34, the second arm rotating portion 35, a second arm 36, a gimbal portion 41, a holder 45, a detector 46, a rotation support portion 47, a drive portion 48, and an attachment/detachment portion 49. The rotating portion 31, the first arm rotating portion 33, the second arm rotating portion 35, and a first joint 42 and a second joint 43 of the gimbal portion 41 are drive-controlled based on control signals inputted from the controller 50. Also, in the present embodiment, the rotation support portion 47 and the drive portion 48 of the surgical instrument 10 are drive-controlled based on control signals inputted from the controller 50.

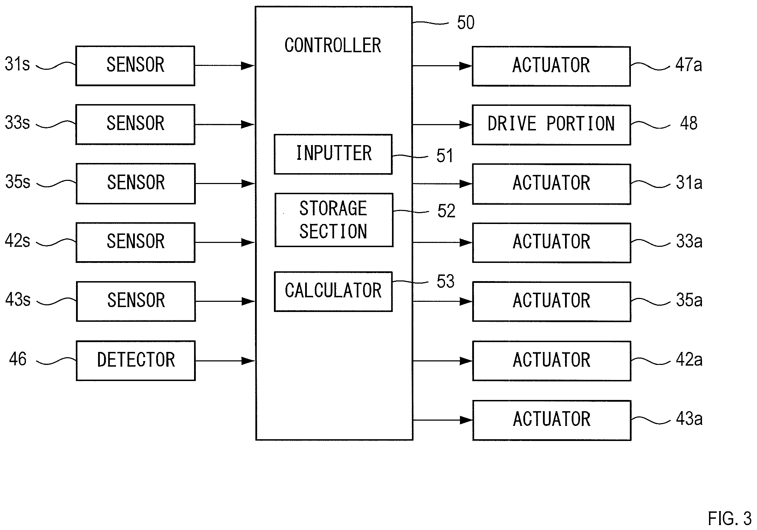

The rotating portion 31 is a joint arranged in a portion where the arm device 30 is fixed to a base 32. The rotating portion 31 is provided with a sensor 31s, shown in FIG. 3, used to detect a phase of the rotating portion 31. It suffices if the rotating portion 31 has a configuration capable of being rotationally driven about a vertically extending rotation axis, and limitation to a specific configuration is not intended.

In the present embodiment, the rotating portion 31 is provided with an actuator 31a that generates a driving force using pressure of gas, such as air, to cause the rotating portion 31 to move rotationally. The rotating portion 31 is rotationally driven by the driving force generated by the actuator 31a.

The first arm rotating portion 33 is arranged between the rotating portion 31 and the first arm 34. The first arm rotating portion 33 has a configuration capable of being rotationally driven about a horizontally extending rotation axis. The first arm rotating portion 33 is provided with a sensor 33s, shown in FIG. 3, used to detect a phase of the first arm rotating portion 33.

In the present embodiment, the first arm rotating portion 33 is provided with an actuator 33a that generates a driving force using pressure of gas, such as air. The first arm rotating portion 33 is rotationally driven by the driving force generated by the actuator 33a.

Although the first arm rotating portion 33 is provided with the actuator 33a in the present embodiment, the first arm rotating portion 33 does not necessarily have to be provided with the actuator 33a.

As shown in FIG. 1, the first arm 34 is arranged between the first arm rotating portion 33 and the second arm rotating portion 35. In the present embodiment, the first arm 34 has a shape extending like a rod; however, its shape is not particularly limited to this shape.

The second arm rotating portion 35 is arranged between the first arm 34 and the second arm 36. The second arm rotating portion 35 has a configuration capable of being rotationally driven about a horizontally extending rotation axis. The second arm rotating portion 35 is provided with a sensor 35s, shown in FIG. 3, used to detect a phase of the second arm rotating portion 35.

In the present embodiment, the second arm rotating portion 35 is provided with an actuator 35a that generates a driving force using pressure of gas, such as air. The second arm rotating portion 35 is rotationally driven by the driving force generated by the actuator 35a.

Although the second arm rotating portion 35 is provided with the actuator 35a in the present embodiment, the second arm rotating portion 35 does not necessarily have to be provided with the actuator 35a.

As shown in FIG. 1, the second arm 36 is arranged between the second arm rotating portion 35 and the gimbal portion 41. In the present embodiment, the second arm 36 has a shape extending like a rod; however, its shape is not particularly limited to this shape.

The gimbal portion 41 is arranged between the second arm 36 and the holder 45. The gimbal portion 41 comprises the first joint 42 and the second joint 43, rotation axes of which intersect with each other, and more preferably, intersect perpendicularly with each other.

The first joint 42 is arranged on a side, of the gimbal portion 41, closer to the second arm 36. The first joint 42 is arranged in an attitude such that a rotation axis thereof extends in a direction in which the second arm 36 extends. The first joint 42 is provided with a sensor 42s, shown in FIG. 3, used to detect a phase of the first joint 42.

The second joint 43 is arranged on a side, of the gimbal portion 41, closer to the holder 45. The second joint 43 is arranged in an attitude such that a rotation axis thereof extends in a direction intersecting, more preferably intersecting perpendicularly, with the direction in which the second arm 36 extends. The second joint 43 is provided with a sensor 43s, shown in FIG. 3, used to detect a phase of the second joint 43.

In the present embodiment, the first joint 42 and the second joint 43 are respectively provided with an actuator 42a and an actuator 43a that generate a driving force using pressure of gas, such as air, to cause the first joint 42 and the second joint 43, respectively, to move rotationally. However, the first joint 42 and the second joint 43 do not necessarily have to be provided with the actuator 42a and the actuator 43a, respectively.

The holder 45 is arranged between the gimbal portion 41 and the rotation support portion 47. It suffices if the holder 45 has a configuration capable of holding the rotation support portion 47 and the drive portion 48, and limitation to a specific configuration is not intended.

The detector 46 is arranged between the holder 45 and the drive portion 48 held by the holder 45. The detector 46 is a force/torque sensor for detecting a force Fw acting on the surgical instrument 10 or on the holder 45 and a torque Mw acting on the surgical instrument 10 or on the holder 45. The torque Mw is also referred to as a "moment". A force torque sensor for detecting a force and a moment can be used as the detector 46, and a detection method and so on are not limited to a particular one.

The force Fw detected by the detector 46 comprises a component of a length-direction force acting in a length direction of the shaft 11, a component of a first intersecting-direction force acting in a first intersecting direction that intersects with the length direction, and a component of a second intersecting-direction force acting in a second intersecting direction that intersects with the length direction and with the first intersecting direction. The torque Mw comprises a component of a length-direction torque acting about the length direction, a component of a first intersecting-direction torque acting about the first intersecting direction, and a component of a second intersecting-direction torque acting about the second intersecting direction. In other words, the detector 46 is a force/torque sensor with six degrees of freedom.

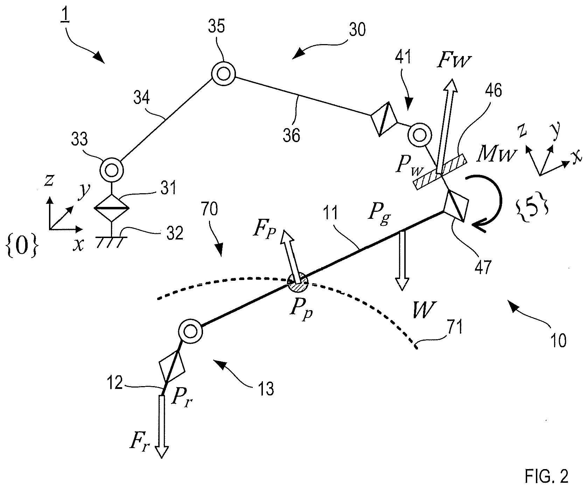

As shown in FIG. 2, in a case where an orthogonal coordinate system {5} is set on the surgical instrument 10, the length direction, the first intersecting direction, and the second intersecting direction described above correspond respectively to an x-direction, a y-direction, and a z-direction in the coordinate system {5}.

A position where the detector 46 is arranged may be between the holder 45 and the drive portion 48 as described above, or may be a position further closer to the shaft 11 of the surgical instrument 10. When an arrangement position of the detector 46 is closer to the shaft 11, improvement of a calculation accuracy of the first external force Fp and the second external force Fr is facilitated as compared with a case where the arrangement position is farther from the shaft 11.

The rotation support portion 47 rotatably supports the drive portion 48 about a central axis of the shaft 11 of the surgical instrument 10. In the state where the surgical instrument 10 is attached to the attachment/detachment portion 49, the rotation support portion 47 rotatably supports the drive portion 48 and the surgical instrument 10 about this central axis. The rotation support portion 47 is provided with an actuator 47a that generates a driving force to control rotation of the rotation support portion 47.

The drive portion 48 is rotatably supported by the rotation support portion 47, and generates the driving force to drive at least one of the distal end part 12 or the bend portion 13 of the surgical instrument 10.

In the present embodiment, a gas-pressure actuator for generating the driving force using pressure of gas, such as air, is arranged in the drive portion 48. In this case, the generated driving force is transmitted to the main body 14 of the surgical instrument 10 via the attachment/detachment portion 49, and is transmitted to at least one of the distal end part 12 or the bend portion 13 via a wire passed through the shaft 11.

A configuration of the drive portion 48 is not particularly limited to a specific configuration. Moreover, a method of generating the driving force may be a method using pressure of gas as described above or may be a method using an electric motor, and limitation to a specific method is not intended.

The attachment/detachment portion 49 is arranged between the drive portion 48 and the surgical instrument 10. The attachment/detachment portion 49 has a configuration allowing for attachment/detachment of the main body 14 of the surgical instrument 10, and also has a configuration allowing for transmission of the driving force from the drive portion 48 to the main body 14. A specific configuration of the attachment/detachment portion 49 is not limited to a particular one.

The controller 50 calculates the first external force that the surgical instrument 10 receives from the trocar 75 and the second external force that the surgical instrument 10 receives from a portion other than the trocar 75. In addition, the controller 50 obtains positions and attitudes of the arm device 30 and the surgical instrument 10 by kinematic calculation, based on information detected by the sensor 31s, the sensor 33s, the sensor 35s, the sensor 42s, and the sensor 43s. The controller 50 outputs a drive command to the actuator 31a and so on based on the obtained positions and attitudes. External force information, such as a value of an external force calculated by a calculator 53 may be incorporated into the drive command to be outputted.

As shown in FIG. 1 and FIG. 3, the controller 50 is an information processor, such as a computer, which comprises a CPU, a ROM, a RAM, an input/output interface, and so on. CPU is an abbreviation for Central Processing Unit. A program stored in a storage device, such as the above-described ROM, causes the CPU, the ROM, the RAM, and the input/output interface to work cooperatively to function as at least an inputter 51, a storage section 52, and the calculator 53.

The inputter 51 receives input of information on the force Fw and the torque Mw that are detected and outputted by the detector 46. Specifically, values of the length-direction force, the first intersecting-direction force, and the second intersecting-direction force, and values of the length-direction torque, the first intersecting-direction torque, and the second intersecting-direction torque are inputted. The inputter 51 also receives input of detection signals from the sensor 31s, the sensor 33s, the sensor 35s, the sensor 42s, and the sensor 43s.

The storage section 52 stores in advance a value of a resistance force $^5Fpx$ acting between the trocar 75 and the shaft 11 of the surgical instrument 10, and a value of a gravity W acting on the surgical instrument 10. A value of weight or mass may be used as the value of the gravity W. The resistance force takes a different value depending on combination of the trocar 75 and the shaft 11. When the trocar 75 and the surgical instrument 10 to be used in the surgical robot system 1 are known, the resistance force is measured or calculated, and is stored in advance in the storage section 52.

The calculator 53 calculates a value of the first external force and a value of the second external force based on the information on the force Fw and the torque Mw inputted to the inputter 51 and on the value of the resistance force stored in advance in the storage section 52. Specific details of calculation of the value of the first external force and the value of the second external force will be described below.

Next, an explanation will be given of calculation of the value of the first external force and the value of the second external force in the surgical robot system 1 having the above-described configuration. First, derivation of equations for calculating the value of the first external force and the value of the second external force will be described with reference to FIG. 2.

To begin with, an equation (1) of equilibrium of translational force as seen from the coordinate system {5} shown in FIG. 2 and an equation (2) of equilibrium of moment about the detector 46 are to be obtained. Here, the coordinate system {5} is an orthogonal coordinate system set on the surgical instrument 10, and the direction in which the shaft 11 extends is set as the x-direction.

[Mathematical 1]

$$F_r + F_p + W + F_w = 0 \tag{1}$$

$$M_w + (P_r - P_w) \times F_r + (P_p - P_w) \times F_p + (P_g - P_w) \times W = 0 \tag{2}$$

In the equations, Fr represents an external force when the second external force to be obtained acts concentratedly on the distal end part 12, which is the forceps. In other words, the second external force to be obtained is assumed to act concentratedly on the distal end part 12 as a resultant force obtained by combining a shaft external force acting also on other portions. Pr represents a position vector of the distal end part 12. Fp represents an external force acting on a portion of the shaft 11 facing the insertion hole, and Pp represents a position vector of the insertion hole. W represents the gravity acting on the surgical instrument 10, and Pg represents a position vector of the center of gravity of the surgical instrument 10. Fw represents a force acting on the holder 45, and is a force detected by the detector 46. Mw represents a moment acting on the holder 45, and is a moment detected by the detector 46. The external force acting on the portion of the shaft 11 facing the insertion hole corresponds to one example of the first external force.

Here, unknowns are only Fp and Fr. Other values can be measured by the sensors or estimated by calculation based on robot forward kinematics. Eliminating Fr from the above equations (1) and (2) yields the following equation (3).
[Mathematical 2]

$$(P_p - P_r) \times F_p = -(P_g - P_r) \times W - (P_w - P_r) \times F_w - M_w \tag{3}$$

As shown in FIG. 2, a coordinate system {0} is set on the arm device 30. The equation (3) is considered in the coordinate system {5}, and an attitude matrix of the coordinate system {0} as seen from the coordinate system {5} is created as below. Using this attitude matrix, the equation (3) is expressed as the following equation (4).

[Mathematical 3]

$$\begin{smallmatrix}5\\0\end{smallmatrix} R$$

[Mathematical 4]

$$\left\{ \begin{smallmatrix}5\\0\end{smallmatrix} R \left(P_p - P_r\right)\right\} \times \left(\begin{smallmatrix}5\\0\end{smallmatrix} RF_p\right) =$$
$$-\left\{\begin{smallmatrix}5\\0\end{smallmatrix} R(P_g - P_r)\right\} \times \left(\begin{smallmatrix}5\\0\end{smallmatrix} RW\right) - \left\{\begin{smallmatrix}5\\0\end{smallmatrix} R(P_w - P_r)\right\} \times \left(\begin{smallmatrix}5\\0\end{smallmatrix} RF_w\right) - \begin{smallmatrix}5\\0\end{smallmatrix} RM_w \qquad (4)$$

Given that the equation (4) is expressed using the following equation and that the right side of the equation (4) is M, the equation (4) is expressed as an equation (5).

[Mathematical 5]

$$\begin{smallmatrix}5\\0\end{smallmatrix} RF_p = {}^5F_p$$

[Mathematical 6]

$$\begin{smallmatrix}5\\0\end{smallmatrix} R\left(P_p - P_r\right) \times {}^5F_p = M \qquad (5)$$

When the equation (5) is expressed using the following equation, the equation (5) is expressed as an equation (6).

[Mathematical 7]

$$\begin{smallmatrix}5\\0\end{smallmatrix} R\left(P_p - P_r\right) = \left[l_{px}, l_{py}, l_{pz}\right]^T$$

[Mathematical 8]

$$\begin{bmatrix} l_{px} \\ l_{py} \\ l_{pz} \end{bmatrix} \times \begin{bmatrix} {}^5F_{px} \\ {}^5F_{py} \\ {}^5F_{pz} \end{bmatrix} = \begin{bmatrix} l_{py}{}^5F_{pz} - l_{pz}{}^5F_{py} \\ l_{pz}{}^5F_{px} - l_{px}{}^5F_{pz} \\ l_{px}{}^5F_{py} - l_{py}{}^5F_{px} \end{bmatrix} = \begin{bmatrix} M_x \\ M_y \\ M_z \end{bmatrix} \qquad (6)$$

The equation (6) is an equation of equilibrium of moment about Pr. The equation (6) is a system of simultaneous equations from a skew-symmetric matrix. Three variables $^5Fpx$, $^5Fpy$, and $^5Fpz$ cannot be solved independently by the equation (6) alone.

Here, $^5Fpx$ is a resistance force acting between the trocar 75 and the shaft 11 of the surgical instrument 10. Thus, a value of $^5Fpx$ can be set in advance as an appropriate condition or value in consideration of a structure of the trocar 75, external force compensation performance of the arm device 30, and so on. Examples of the value of $^5Fpx$ may include the following three conditions. In the following conditions, f represents a constant, and $\mu$ represents a coefficient of friction.

[Mathematical 9]

$$^{5}F_{px} = 0$$

$$^{5}F_{px} = f$$

$$^{5}F_{px} = \mu N$$

where

$$N = \sqrt{^{5}F_{py}{}^{2} + {}^{5}F_{pz}{}^{2}}$$

For example, when the condition $^{5}Fpx=0$ is used, solving for $^{5}Fpy$ and $^{5}Fpz$ of the equation (6) yields the following equations (7) and (8).

[Mathematical 10]

$$^{5}F_{py} = \frac{M_{z}}{l_{px}} \tag{7}$$

$$^{5}F_{pz} = -\frac{M_{y}}{l_{px}} \tag{8}$$

Further, solving for Fry and Frz using a relation between the above equations (7) and (8) and the equation (1) results as shown in the following equations (9) and (10).

[Mathematical 11]

$$F_{ry} = -F_{py} - W_{y} - F_{wy} \tag{9}$$

$$F_{rz} = -F_{pz} - W_{z} - F_{wz} \tag{10}$$

An external force Frx parallel to the shaft 11 generated at the distal end part 12 can be obtained from the following relational equation (11) of a resultant force in terms of an x-direction component of the detector 46. The equation (11) shows an equation with the term of Fpx left, so as to facilitate understanding when a condition other than $^{5}Fpx=0$ is used.
[Mathematical 12]

$$F_{wx} = -F_{rx} - F_{px} - W_{x} \tag{11}$$

When the condition $^{5}Fpx=f$ is used or when the condition $^{5}Fpx=\mu N$ is used, too, solving for $^{5}Fpy$ and $^{5}Fpz$ of the equation (6) yields equations for obtaining respective values. As for Fry, Frz, and Frx, too, equations for obtaining respective values are yielded in a manner similar to the above.
The methods of obtaining the values of $^{5}Fpy$, $^{5}Fpz$, Fry, Frz, and Frx may be those using an analytical approach based on deformation of the equations as described above, or may be those using simultaneous equations approach, such as a numerical analysis method using a computer.

Next, an explanation will be given of calculation of the value of the first external force and the value of the second external force in the surgical robot system 1. Here, Fp as described above corresponds to the first external force, and Fr as described above corresponds to one example of the second external force. The calculator 53 in the controller 50 performs an operation of calculating the above-described Fp and Fr.

The controller 50 acquires, from the storage section 52, the value of the gravity W acting on the surgical instrument 10 to be used in calculating Fp and Fr, and also performs a process of acquiring, from the detector 46, the force Fw acting on the holder 45 and the moment Mw acting on the holder 45.

Further, the controller 50 performs an operation of obtaining position vectors of Pr, Pp, Pg, and Pw to be used in calculating Fp and Fr. When performing the operation, the controller 50 performs an arithmetic process for acquiring detection signals to be used in calculating the position vectors and control signals for controlling an attitude of the bend portion 13. The detection signals are outputted from the sensor 31s, the sensor 33s, the sensor 35s, the sensor 42s, and the sensor 43s.

As for mathematical equations used in obtaining the position vectors and as for size information including the length and the shape of the first arm 34 and so on in the arm device 30, those stored in advance in the storage section 52 are used.

In the above-described embodiment, in obtaining the position vectors of Pr, Pp, Pg, and Pw, the sensor 31s, the sensor 33s, the sensor 35s, the sensor 42s, and the sensor 43s for detecting the respective phases are used. However, the control signals for controlling the attitude of the arm device 30 may be used instead of outputs of the sensors for detecting the respective phases.

Similarly, although the control signals for controlling the attitude of the bend portion 13 are used in the above-described embodiment, outputs of the sensors for detecting the attitude of the bend portion 13 may be used instead of the control signals.

As for the equations used in obtaining the position vectors and as for parameters included in the mathematical equations, various equations and parameters can be used according to the types and the shapes of the arm device 30 and the surgical instrument 10, and limitation to those in the present embodiment is not intended.

Subsequently, the calculator 53 in the controller 50 performs an operation of calculating Fp and Fr using the position vectors of Pr, Pp, Pg, and Pw, the gravity W, the force Fw, the moment Mw, the above-described equations (7), (8), (9), (10), and (11), and so on.

The surgical robot system 1, the controller 50, and the program each configured as described above, make it possible to calculate the value of the first external force Fp that the surgical instrument 10 receives from the trocar 75 and the value of the second external force Fr that the surgical instrument 10 receives from a portion other than the trocar 75, based on values of various external forces and torques detected by the detector 46 and on the value of the resistance force ${}^5Fpx$ acting between the trocar 75 and the shaft 11.

Since the detector 46 for detecting the values of various external forces and torques is arranged at a position not where the shaft 11 is present but where the arm device 30 is present, an attempt to reduce the size of the surgical instrument 10 is facilitated as compared with a case where the sensor is arranged at the distal end part of the surgical instrument 10.

As compared with a case where, for example, the detector 46 is arranged closer to the distal end part 12 than the drive portion 48 is, arranging the detector 46 between the drive portion 48 and the holder 45 makes it less likely for the detector 46 to detect a force and a torque caused by operation of the drive portion 48. In other words, improvement in accuracy of detection by the detector 46 is facilitated.

Each component of the external force can be obtained independently based on various information outputted from the detector 46, which is the force/torque sensor with six degrees of freedom. Thus, theoretically speaking, there occurs no case where the external force acting on the distal end part 12, which is the forceps, and/or on the trocar 75 is undercalculated.

The surgical robot system 1 is controlled using the calculated value of the first external force Fp and the calculated value of the second external force Fr. This facilitates effective utilization as force feedback and safety feature by appropriately setting thresholds for the control even when some uncertainty is contained in the gravity W acting on the surgical instrument 10, a frictional force Fpx of the trocar 75, and so on.

For example, it becomes possible to achieve an operation like maintaining the position and the attitude of the distal end part 12 without yielding to the external force acting on the shaft 11 of the surgical instrument 10 while suppressing the first external force Fp that the surgical instrument 10 receives from the trocar 75 below a certain level.

It is possible to detect the first external force Fp that the surgical instrument 10 receives from the trocar 75 and the second external force Fr that the surgical instrument 10 receives from a portion other than the trocar 75 in a significantly distinguishable manner. Thus, enhancement of the safety in and out of the visual field during surgery is facilitated by presenting the first external force Fp and the second external force Fr to a manipulator of the surgical robot system 1 by some means.

[Second Embodiment]

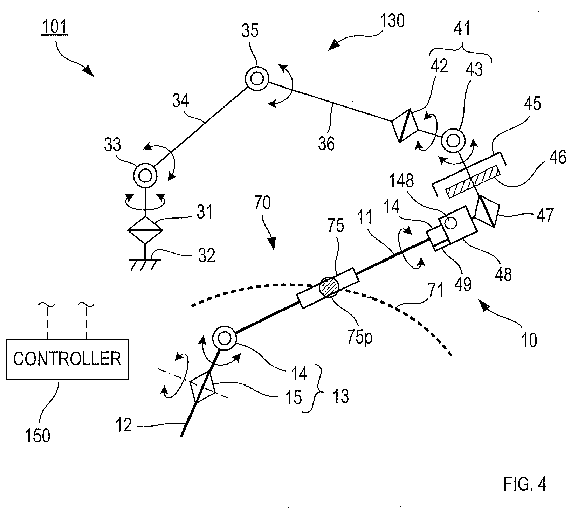

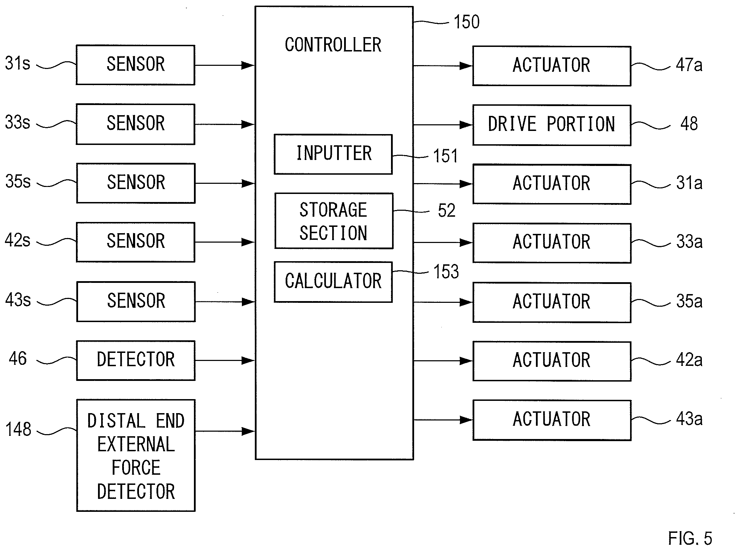

Next, a surgical robot system according to a second embodiment of the present disclosure will be described with reference to FIG. 4 and FIG. 5. A basic configuration of the surgical robot system of the present embodiment is similar to that of the first embodiment; however, the method for obtaining the external force received by the distal end part of the surgical instrument is different from that of the first embodiment. Thus, in the present embodiment, an explanation will be given of the method for obtaining the external force received by the distal end part of the surgical instrument with reference to FIG. 4 and FIG. 5, and explanation of others will be omitted.

As shown in FIG. 4, a surgical robot system 101 of the present embodiment comprises the surgical instrument 10, an arm device 130, and a controller 150. The controller 150 corresponds to one example of an element as an external force estimation device.

The arm device 130 comprises the rotating portion 31, the first arm rotating portion 33, the first arm 34, the second arm rotating portion 35, the second arm 36, the gimbal portion 41, the holder 45, the detector 46, the rotation support portion 47, the drive portion 48, the attachment/detachment portion 49, and a distal end external force detector 148. The distal end external force detector 148 is a sensor for detecting a value of a distal end external force Ft, which is an external force received by the distal end part 12 of the surgical instrument 10.

In the present embodiment, in a case where the drive portion 48 comprises a gas-pressure actuator for generating a driving force that drives the distal end part 12 and the bend portion 13 using pressure of gas, such as air, the distal end external force detector 148 includes a sensor for detecting displacement of a piston and so on in the gas-pressure actuator and a sensor for detecting pressure of gas within the cylinder.

It suffices if the distal end external force detector 148 detects the external force Ft acting on the distal end part 12, and it is not intended to limit its arrangement position and a detection method. For example, the distal end external force detector 148 may be a sensor that is arranged in the neighborhood of the distal end part 12 in the surgical instrument 10 to detect the external force Ft acting on the distal end part 12.

As shown in FIG. 4 and FIG. 5, the controller 150 is an information processor, such as a computer, which comprises a CPU, a ROM, a RAM, an input/output interface, and so on. CPU is an abbreviation for Central Processing Unit.

A program stored in a storage device, such as the above-described ROM, causes the CPU, the ROM, the RAM, and the input/output interface to work cooperatively to function as at least an inputter 151, the storage section 52, and a calculator 153.

As compared with the inputter 51 of the first embodiment, the inputter 151 further receives input of information on the distal end external force Ft that is applied to the distal end part 12 and detected by the distal end external force detector 148.

The calculator 153 calculates a value of the first external force Fp and a value of the second external force Fr based on the information on the force Fw and the torque Mw inputted to the inputter 151, on the information on the distal end external force Ft, and on the value of the resistance force stored in advance in the storage section 52.

The calculator 153 of the present embodiment performs an operation similar to that in the first embodiment when calculating the value of the first external force Fp and the value of the second external force Fr. When calculating a value of the distal end external force Ft acting on the distal end part 12, the calculator 153 performs an operation based on the information on the distal end external force Ft detected and outputted by the distal end external force detector 148. Furthermore, calculation of the shaft external force, which is an external force received from a portion other than the distal end part 12 and the trocar 75, is performed using the calculated second external force Fr and the calculated distal end external force Ft acting on the distal end part 12. Given that a resultant force of the shaft external force is Fs, Fs can be obtained using the following equation.

[Mathematical 13]

$$F_s = F_r - F_t$$

The above-described configuration with the distal end external force detector 148 provided thereto enables the calculator 153 to calculate a value of the shaft external force, which is an external force in the shaft 11 received from a portion other than the distal end part 12 and the trocar 75. Examples of the shaft external force may include a force applied due to contact of a midsection of the shaft 11 with an organ or the like of the patient 70, which is caused by relative movement of the surgical instrument 10 with respect to the patient 70.

In addition, use of the distal end external force Ft obtained by the distal end external force detector 148 as a reference signal facilitates further improvement in accuracy of the first external force Fp and the second external force Fr to be calculated, as compared with a case where the distal end external force detector 148 is not used.

[Third Embodiment]

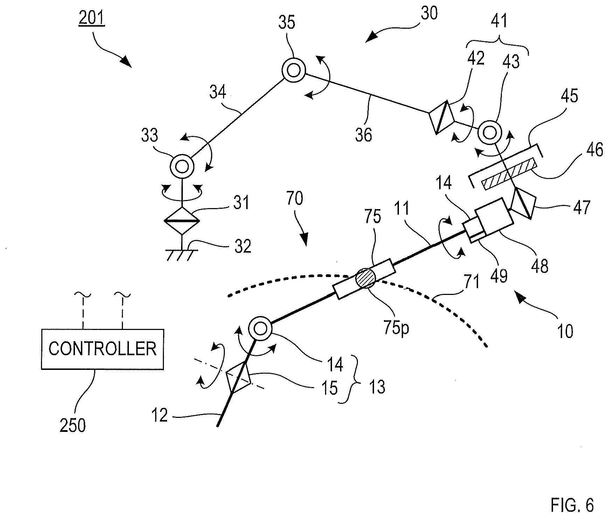

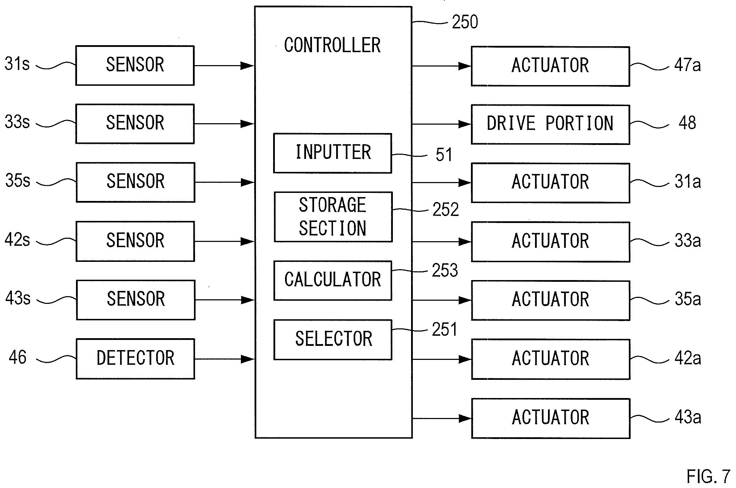

Next, a surgical robot system according to a third embodiment of the present disclosure will be described with reference to FIG. 6 and FIG. 7. A basic configuration of the surgical robot system of the present embodiment is similar to that of the first embodiment; however, the present embodiment differs from the first embodiment in that the surgical robot system is adaptable to a different type of trocar. Thus, in the present embodiment, an explanation will be given of details of the adaptability to a different type of trocar with reference to FIG. 6 and FIG. 7, and explanation of other configuration and so on will be omitted.

As shown in FIG. 6, a surgical robot system 201 of a embodimen of the present invention comprises the surgical instrument 10, the arm device 30, and a controller 250. The controller 250 corresponds to one example of an element as an external force estimation device.

As shown in FIG. 6 and FIG. 7, the controller 250 is an information processor, such as a computer, which comprises a CPU, a ROM, a RAM, an input/output interface, and so on. CPU is an abbreviation for Central Processing Unit. A program stored in a storage device, such as the above-described ROM, causes the CPU, the ROM, the RAM, and the input/output interface to work cooperatively to function as at least the inputter 51, a selector 251, a storage section 252, and a calculator 253.

The selector 251 is used to identify the trocar 75 to be used in the surgical robot system 1. In the present embodiment, multiple types of the trocar 75 are used in the surgical robot system 1, and information for identifying the trocar 75 of each type is acquired.

Examples of such a case may include a case where the selector 251 displays the types of the trocar 75 used in the surgical robot system 1 and performs identification by designating a type of the trocar 75 to be used from among the displayed types of the trocar 75.

Further example may be included in which the trocar 75 to be used is identified by inputting model number, serial number, or the like indicated on the trocar 75 to the selector 251, and limitation to a specific method of performing identification is not intended.

The storage section 252 stores in advance information for identifying the trocar 75 to be used in the surgical robot system 1, a value of the resistance force $^5Fpx$ acting between the trocar 75 and the shaft 11 of the surgical instrument 10, and a value of the gravity W acting on the surgical instrument 10. A value of weight may be used as the gravity W. The information for identifying the trocar 75 and the value of the resistance force $^5Fpx$ in such trocar 75 are stored in an associated manner.

The calculator 253 calculates a value of the first external force Fp and a value of the second external force Fr based on the information on the force Fw and the torque Mw inputted to the inputter 51 and based on the value of the resistance force stored in advance in the storage section 252. The calculation in the calculator 253 differs from that in the first embodiment in that the information for identifying the trocar 75 selected by the selector 251 and the value of the resistance force $^5Fpx$ associated therewith are acquired from the storage section 252 to thereby perform an operation. The calculation other than this point is similar to that in the first embodiment.

The above-described configuration with the storage section 252 and the selector 251 provided thereto makes it possible to calculate the value of the first external force Fp and the value of the second external force Fr using the resistance force $^5Fpx$ corresponding to the trocar 75 to be used, even when the trocar 75 to be used is changed to the trocar 75 different in the resistance force $^5Fpx$ acting between the trocar 75 and the shaft 11. This results in facilitating reduction of deterioration in accuracy of the value of the first external force Fp and the value of the second external force Fr to be calculated, as compared with a case where the resistance force $^5Fpx$ corresponding to the trocar 75 is not used.

For example, in the above-described embodiments, the distal end part 12 provided to the distal end of the shaft 11 is the forceps. However, the distal end part 12 is not limited to the forceps, and may be other instruments used in endoscope surgery and so on.

Examples of the endoscopic surgery to which the surgical robot system, the external force estimation device, and the program of the present disclosure are applied may include endoscopic surgeries in general in which the surgical instrument 10 is inserted into the body of the patient 70. From among the endoscopic surgeries, gastroenterological surgeries on large organ, such as digestive organ, in which an external force is likely to be applied to the surgical instrument 10, thoracic surgeries in which the surgical instrument 10 is inserted in between costal bones, and surgeries for obese patients with a thick abdominal wall are endoscopic surgeries in which the surgical robot system of the present disclosure is used in a preferred manner.

**Claims**

**1.** An external force estimation device (250) comprising:

an inputter (51, 151) configured to receive, from a detector (46), input of detection signals indicating values of forces and torques detected by the detector (46), the detector being arranged at a position where a shaft (11) of a surgical instrument (10) is not present but where an arm portion (30, 130) is present, the shaft (11) being formed in a long shape and inserted through a trocar (75) placed in a subject (70), the arm portion (30, 130) being configured to grasp the surgical instrument (10), wherein the arm portion can move with respect to the subject (70), the values being values of:

a length-direction force acting on the surgical instrument (10) in a length direction of the shaft (11), a length-direction torque acting on the surgical instrument (10) about the length direction, a first intersecting-direction force acting on the surgical instrument (10) in a first intersecting direction that intersects with the length direction,
a first intersecting-direction torque acting on the surgical instrument (10) about the first intersecting direction, a second intersecting-direction force acting on the surgical instrument (10) in a second intersecting direction that intersects with the length direction and with the first intersecting direction, and a second intersecting-direction torque acting on the surgical instrument (10) about the second intersecting direction; wherein the inputter further receives a position and attitude of the instrument;

a calculator (53, 153) configured to calculate a value of a first external force that the surgical instrument (10) receives from the trocar (75) and a value of a second external force that the surgical instrument (10) receives from a portion other than the trocar (75), based on the values of the length-direction force, the first intersecting-direction force, the second intersecting-direction force, the length-direction torque, the first intersecting-direction torque, the second intersecting-direction torque, the position and attitude of the instrument and on a value of a resistance force acting between the trocar (75) and the shaft (11);a storage section (252) in which values of resistance forces each acting between the trocar (75) of a different type and the shaft (11) are stored in association with types of the trocar (75); and
a selector (251) configured to identify the type of the trocar (75),
wherein the calculator (53, 153) acquires, from the storage section (252), the value of the resistance force for the type of the trocar (75) identified by the selector (251), and calculates the first external force and the second external force using the value of the resistance force acquired.

**2.** A surgical robot system (201) provided with the external force estimation device (250) according to claim 1, comprising:

the surgical instrument (10);
the arm portion (30, 130);
the detector (46); and
the external force estimation device (250).

**3.** The surgical robot system (201) according to claim 2, further comprising:

a drive portion (48) configured to drive a distal end part (12) of the shaft (11) of the surgical instrument (10), wherein the arm portion (30, 130) comprises a holder (45) configured to hold the surgical instrument (10), and wherein the detector (46) is arranged between the drive portion (48) and the holder (45).

**4.** The surgical robot system (201) according to claim 3, further comprising:

a distal end external force detector (148) configured to detect a value of a distal end external force, the distal end external force being an external force that the distal end part (12) receives,
wherein the calculator (53, 153) calculates a value of a shaft external force, the shaft external force being an external force in the shaft (11) received from a portion other than the distal end part (12) and the trocar (75), based on the value of the second external force and the value of the distal end external force.

**5.** A program configured to cause a computer coupled to the external force estimation device of claim 1 to perform:

an input function (51, 151) of receiving, from a detector (46), input of detection signals indicating values of forces and torques detected by the detector (46), the detector being arranged at a position where a shaft (11) of a surgical instrument (10) is not present but where an arm portion (30, 130) is present, the shaft (11) being formed in a long shape and inserted through a trocar (75) placed in a subject (70), the arm portion (30, 130) being configured to grasp the surgical instrument (10), wherein the arm portion can move with respect to the subject (70), the values being values of:

a length-direction force acting on the surgical instrument (10) in a length direction of the shaft (11),
a length-direction torque acting on the surgical instrument (10) about the length direction,
a first intersecting-direction force acting on the surgical instrument (10) in a first intersecting direction that intersects with the length direction,
a first intersecting-direction torque acting on the surgical instrument (10) about the first intersecting direction,
a second intersecting-direction force acting on the surgical instrument (10) in a second intersecting direction that intersects with the length direction and with the first intersecting direction, and
a second intersecting-direction torque acting on the surgical instrument (10) about the second intersecting direction; wherein the input function further receives a position and attitude of the instrument;

a calculation function (53, 153) of calculating a value of a first external force that the surgical instrument (10) receives from the trocar (75) and a value of a second external force that the surgical instrument (10) receives from a portion other than the trocar (75), based on the values of the length-direction force, the first intersecting-direction force, the second intersecting-direction force, the length-direction torque, the first intersecting-direction torque, the second intersecting-direction torque, the position and attitude of the instrument and on a value of a resistance force acting between the trocar (75) and the shaft (11); and
a selection function (251) of identifying a type of the trocar (75),
wherein the calculation function (53, 153) acquires, from a storage section (252), the value of the resistance force for the type of the trocar (75) identified by the selection function (251), and calculates the first external force and the second external force using the value of the resistance force acquired, and
wherein the storage section (252) stores values of resistance forces each acting between the trocar (75) of a different type and the shaft (11) in association with the types of the trocar (75).


**Patentansprüche**

1.  Außenkraft-Schätzvorrichtung (250), enthaltend:

eine Eingabevorrichtung (51, 151), die konfiguriert ist, von einem Detektor (46) eine Eingabe von Detektionssignalen zu empfangen, die Werte von von dem Detektor (46) detektierten Kräften und Drehmomenten anzeigen, wobei der Detektor an einer Position angeordnet ist, an der ein Schaft (11) eines chirurgischen Instruments (10) nicht vorhanden ist, aber an der ein Armabschnitt (30, 130) vorhanden ist, wobei der Schaft (11) in einer langen Form ausgebildet ist und durch einen Trokar (75) eingeführt wird, der in einem Subjekt (70) platziert ist, wobei der Armabschnitt (30, 130) konfiguriert ist, das chirurgische Instrument (10) zu greifen, wobei sich der Armabschnitt in Bezug auf das Subjekt (70) bewegen kann, wobei die Werte Werte sind von:

einer Längsrichtungskraft, die auf das chirurgische Instrument (10) in eine Längsrichtung des Schafts (11) einwirkt,
einem Längsrichtungsdrehmoment, das auf das chirurgische Instrument (10) um die Längsrichtung einwirkt,
einer ersten Schnittrichtungskraft, die auf das chirurgische Instrument (10) in einer ersten Schnittrichtung einwirkt, die sich mit der Längsrichtung schneidet,
einem ersten Schnittrichtungsdrehmoment, das auf das chirurgische Instrument (10) um die erste Schnittrichtung einwirkt,
einer zweiten Schnittrichtungskraft, die auf das chirurgische Instrument (10) in eine zweite Schnittrichtung einwirkt, die sich mit der Längsrichtung und mit der ersten Schnittrichtung schneidet, und
einem zweiten Schnittrichtungsdrehmoment, das auf das chirurgische Instrument (10) um die zweite Schnittrichtung einwirkt; wobei die Eingabevorrichtung ferner eine Position und Lage des Instruments empfängt;

einen Rechner (53, 153), der konfiguriert ist, einen Wert einer ersten Außenkraft, die das chirurgische Instrument (10) von dem Trokar (75) empfängt, und einen Wert einer zweiten Außenkraft, die das chirurgische Instrument (10) von einem anderen Abschnitt als dem Trokar (75) empfängt, zu berechnen, basierend auf den Werten der Längsrichtungskraft, der ersten Schnittrichtungskraft, der zweiten Schnittrichtungskraft, des Längsrichtungsdrehmoments, des ersten Schnittrichtungsdrehmoments, des zweiten Schnittrichtungsdrehmoments, der Position und Lage des Instruments und einem Wert einer Widerstandskraft, die zwischen dem Trokar (75) und dem Schaft (11) einwirkt; einen Speicherabschnitt (252), in dem Werte von Widerstandskräften, die jeweils zwischen dem Trokar (75) eines anderen Typs und dem Schaft (11) einwirken, in Verbindung mit Typen des Trokars (75) gespeichert sind; und

einen Selektor (251), der konfiguriert ist, den Typ des Trokars (75) zu identifizieren,

wobei der Rechner (53, 153) aus dem Speicherabschnitt (252) den Wert der Widerstandskraft für den Typ des Trokars (75) bezieht, der durch den Selektor (251) identifiziert wird, und die erste Außenkraft und die zweite Außenkraft unter Verwendung des Wertes der bezogenen Widerstandskraft berechnet.

**2.** Chirurgisches Robotersystem (201), bereitgestellt mit der Außenkraft-Schätzvorrichtung (250) nach Anspruch 1, enthaltend:

das chirurgische Instrument (10);
den Armabschnitt (30, 130);
den Detektor (46); und
die Außenkraft-Schätzvorrichtung (250).

**3.** Chirurgisches Robotersystem (201) nach Anspruch 2, ferner enthaltend:

einen Antriebsabschnitt (48), der konfiguriert ist, ein Distalendeteil (12) des Schaftes (11) des chirurgischen Instruments (10) anzutreiben,

wobei der Armabschnitt (30, 130) eine Halterung (45) aufweist, die konfiguriert ist, das chirurgische Instrument (10) zu halten, und

wobei der Detektor (46) zwischen dem Antriebsabschnitt (48) und der Halterung (45) angeordnet ist.

**4.** Chirurgisches Robotersystem (201) nach Anspruch 3, ferner enthaltend:

einen Distalende-Außenkraftdetektor (148), der konfiguriert ist, einen Wert einer Außenkraft des Distalendes zu detektieren, wobei die Distalende-Außenkraft eine Außenkraft ist, die das Distalendeteil (12) empfängt,

wobei der Rechner (53, 153) einen Wert einer Schaft-Außenkraft berechnet, wobei die Schaft-Außenkraft eine Außenkraft in dem Schaft (11) ist, die von einem anderen Abschnitt als dem Distalendeteil (12) und dem Trokar (75) empfangen wird, basierend auf dem Wert der zweiten Außenkraft und dem Wert der Distalende-Außenkraft.

**5.** Programm, das konfiguriert ist, einen Computer, der mit der Außenkraft-Schätzvorrichtung nach Anspruch 1 gekoppelt ist, zu veranlassen, auszuführen:

eine Eingangsfunktion (51, 151) zum Empfangen von Detektionssignalen von einem Detektor (46), die Werte von von dem Detektor (46) detektierten Kräften und Drehmomenten anzeigen, wobei der Detektor an einer Position angeordnet ist, an der ein Schaft (11) eines chirurgischen Instruments (10) nicht vorhanden ist, sondern ein Armabschnitt (30, 130) vorhanden ist, wobei der Schaft (11) in einer langen Form ausgebildet und durch einen Trokar (75) eingeführt wird, der in einem Subjekt (70) platziert ist, wobei der Armabschnitt (30, 130) konfiguriert ist, das chirurgische Instrument (10) zu greifen, wobei sich der Armabschnitt in Bezug auf das Subjekt (70) bewegen kann, wobei die Werte Werte sind von:

einer Längsrichtungskraft, die auf das chirurgische Instrument (10) in eine Längsrichtung des Schafts (11) einwirkt,

einem Längsrichtungsdrehmoment, das auf das chirurgische Instrument (10) um die Längsrichtung einwirkt,

einer ersten Schnittrichtungskraft, die auf das chirurgische Instrument (10) in einer ersten Schnittrichtung einwirkt, die sich mit der Längsrichtung schneidet,

einem ersten Schnittrichtungsdrehmoment, das auf das chirurgische Instrument (10) um die erste Schnittrichtung einwirkt,

einer zweiten Schnittrichtungskraft, die auf das chirurgische Instrument (10) in eine zweite Schnittrichtung einwirkt, die sich mit der Längsrichtung und mit der ersten Schnittrichtung schneidet, und

einem zweiten Schnittrichtungsdrehmoment, das auf das chirurgische Instrument (10) um die zweite Schnittrichtung einwirkt; wobei die Eingabevorrichtung ferner eine Position und Lage des Instruments empfängt;

eine Berechnungsfunktion (53, 153) zum Berechnen eines Werts einer ersten Außenkraft, die das chirurgische Instrument (10) von dem Trokar (75) empfängt, und eines Werts einer zweiten Außenkraft, die das chirurgische Instrument (10) von einem anderen Abschnitt als dem Trokar (75) empfängt, basierend auf den Werten der Längsrichtungskraft, der ersten Schnittrichtungskraft, der zweiten Schnittrichtungskraft, des Längsrichtungsdrehmoments, des ersten Schnittrichtungsdrehmoments, des zweiten Schnittrichtungsdrehmoments, der Position und Lage des Instruments und einem Wert einer Widerstandskraft, die zwischen dem Trokar (75) und dem Schaft (11) einwirkt;
eine Selektionsfunktion (251) zum Identifizieren eines Typs des Trokars (75),
wobei die Berechnungsfunktion (53, 153) aus dem Speicherabschnitt (252) den Wert der Widerstandskraft für den Typ des Trokars (75) bezieht, der durch die Selektionsfunktion (251) identifiziert wird, und die erste Außenkraft und die zweite Außenkraft unter Verwendung des Wertes der bezogenen Widerstandskraft berechnet, und wobei der Speicherabschnitt (252) Werte von Widerstandskräften, die jeweils zwischen dem Trokar (75) eines anderen Typs und dem Schaft (11) einwirken, in Verbindung mit den Typen des Trokars (75) speichert.

## Revendications

1. Un dispositif d'estimation de force externe (250) comprenant :

   un dispositif d'entrée (51, 151) configurée pour recevoir, à partir d'un détecteur (46), une entrée de signaux de détection indiquant les valeurs des forces et des couples détectées par le détecteur (46), le détecteur étant disposé à une position où une tige (11) d'un instrument chirurgical (10) n'est pas présent mais où une partie de bras (30, 130) est présente, la tige (11) étant formé en forme de la longueur et inséré par un trocart (75) placé dans un sujet (70), la partie de bras (30, 130) étant configurée pour saisir l'instrument chirurgical (10), dans lequel la partie de bras peut se déplacer par rapport au sujet (70), les valeurs étant les valeurs de :

   une force de direction de la longueur agissant sur l'instrument chirurgical (10) dans une direction de la longueur de la tige (11),
   un couple de direction de la longueur agissant sur l'instrument chirurgical (10) autour de la direction de la longueur,
   une première force de direction d'intersection agissant sur l'instrument chirurgical (10) dans un première direction d'intersection qui recoupe avec la direction de la longueur,
   un premier couple de direction d'intersection agissant sur l'instrument chirurgical (10) autour de la première direction d'intersection,
   une deuxième force de direction d'intersection agissant sur l'instrument chirurgical (10) dans une deuxième direction d'intersection qui recoupe avec la direction de la longueur et avec la première direction d'intersection, et
   un deuxième couple de direction d'intersection agissant sur l'instrument chirurgical (10) autour de la deuxième direction d'intersection; dans lequel le dispositif d'entrée reçoit en outre une position et une attitude de l'instrument ;

   un calculateur (53, 153) configuré pour calculer la valeur d'une première force externe que l'instrument chirurgical (10) reçoit du trocart (75) et la valeur d'une deuxième force externe que l'instrument chirurgical (10) reçoit d'une partie autre que le trocart (75), sur la base des valeurs de la force de direction de la longueur, de la première force de direction d'intersection, de la deuxième force de direction d'intersection, du couple de direction de la longueur, du premier couple de direction d'intersection, du deuxième couple de direction d'intersection, de la position et de l'attitude de l'instrument et de la valeur d'une force de résistance agissant entre le trocart (75) et la tige (11) ; une section de stockage (252), dans laquelle les valeurs des forces de résistance agissant chacune entre le trocart (75) d'un type différent et la tige (11) sont stockées en association avec les types du trocart (75) ; et
   un sélecteur (251) configuré pour identifier le type de trocart (75),
   dans lequel le calculateur (53, 153) acquiert, à partir de la section de stockage (252), la valeur de la force de résistance pour le type de trocart (75) identifié par le sélecteur (251), et calcule la première force externe et la deuxième force externe en utilisant la valeur de la force de résistance acquise.

2. Un système de robot chirurgical (201) muni d'un dispositif d'estimation de force externe (250) selon la revendication 1, comprenant :

   l'instrument chirurgical (10) ;
   la partie de bras (30, 130) ;
   le détecteur (46) ; et
   le dispositif d'estimation de force externe (250).

**3.** Le système de robot chirurgical (201) selon la revendication 2, comprenant en outre :

une partie d'entraînement (48) configurée pour entraîner une partie à extrémité distale (12) de la tige (11) de l'instrument chirurgical (10),
dans lequel la partie de bras (30, 130) comprend un support (45) configuré pour tenir l'instrument chirurgical (10), et
dans lequel le détecteur (46) est disposé entre la partie d'entraînement (48) et le support (45).

**4.** Le système de robot chirurgical (201) selon la revendication 3, comprenant en outre :

un détecteur de force externe à extrémité distale (148) configuré pour détecter une valeur d'une force externe à extrémité distale, la force externe à extrémité distale étant une force externe que la partie à extrémité distale (12) reçoit,
dans lequel le calculateur (53, 153) calcule une valeur de la force externe de la tige, la force externe de la tige étant une force externe dans la tige (11) reçue d'une partie autre que la partie à extrémité distale (12) et le trocart (75), sur la base de la valeur de la deuxième force externe et de la valeur de la force externe à extrémité distale.

**5.** Un programme configuré pour faire qu'un ordinateur couplé au dispositif d'estimation de force externe selon la revendication 1 exécute :

une fonction d'entrée (51, 151) pour recevoir, à partir d'un détecteur (46), des signaux de détection indiquant des valeurs des forces et des couples détectées par le détecteur (46), le détecteur étant disposé à une position où une tige (11) d'un instrument chirurgical (10) n'est pas présente mais où une partie de bras (30, 130) est présente, la tige (11) étant formée en forme de la longueur et insérée par un trocart (75) placé dans un sujet (70), la partie de bras (30, 130) étant configurée pour saisir l'instrument chirurgical (10), dans lequel la partie de bras peut se déplacer par rapport au sujet (70), les valeurs étant les valeurs de :

une force de direction de la longueur agissant sur l'instrument chirurgical (10) dans une direction de la longueur de la tige (11),
un couple de direction de la longueur agissant sur l'instrument chirurgical (10) autour de la direction de la longueur,
une première force de direction d'intersection agissant sur l'instrument chirurgical (10) dans un première direction d'intersection qui recoupe avec la direction de la longueur,
un premier couple de direction d'intersection agissant sur l'instrument chirurgical (10) autour de la première direction d'intersection,
une deuxième force de direction d'intersection agissant sur l'instrument chirurgical (10) dans une deuxième direction d'intersection qui recoupe avec la direction de la longueur et avec la première direction d'intersection, et
un deuxième couple de direction d'intersection agissant sur l'instrument chirurgical (10) autour de la deuxième direction d'intersection; dans lequel la fonction d'entrée reçoit en outre une position et une attitude de l'instrument ;

une fonction de calcul (53, 153) pour calculer une valeur d'une première force externe que l'instrument chirurgical (10) reçoit du trocart (75) et une valeur d'une deuxième force externe que l'instrument chirurgical (10) reçoit d'une partie autre que le trocart (75), sur la base des valeurs de la force de direction de la longueur, de la première force de direction d'intersection, de la deuxième force de direction d'intersection, du couple de direction de la longueur, du premier couple de direction d'intersection, du deuxième couple de direction d'intersection, de la position et de l'attitude de l'instrument et de la valeur d'une force de résistance agissant entre le trocart (75) et la tige (11) ;
une fonction de sélection (251) pour identifier un type de trocart (75),
dans lequel la fonction de calcul (53, 153) acquiert, à partir d'une section de stockage (252), la valeur de la force de résistance pour le type de trocart (75) identifié par la fonction de sélection (251), et calcule la première force externe et la deuxième force externe en utilisant la valeur de la force de résistance acquise, et
dans lequel la section de stockage (252) stocke des valeurs des forces de résistance agissant chacune entre le trocart (75) d'un type différent et la tige (11) en association avec les types du trocart (75).

FIG. 1

FIG. 2

FIG. 3

FIG. 4

FIG. 5

FIG. 6

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009522016 A
- EP 1915963 A1
- EP 3205459 A1
- WO 2015079775 A1
- WO 2019056871 A1

**Non-patent literature cited in the description**

- Active wrists endoscope navigation in robotized laparoscopic surgery. **BAUZANO E. et al.** MECHATRONICS, 2009. ICM 2009. IEEE INTERNATIONAL CONFERENCE ON. IEEE, 14 April 2009, 1-6
- Safe teleoperation of a laparoscope holder with dynamic precision but low stiffness. **MAGO JESUS et al.** 2019 INTERNATIONAL CONFERENCE ON ROBOTICS AND AUTOMATION (ICRA). IEEE, 20 May 2019, 2693-2699
- A 3-DOF sensor to estimate the force applied to the tip of a surgical instrument. **KIM SUYONG et al.** 18TH INTERNATIONAL CONFERENCE ON ADVANCED ROBOTICS (ICAR). IEEE, 10 July 2017, 143-148
- A method to estimate the axial force applied to a surgical instrument tip considering the effect of the gravity. **KIM SUYONG et al.** 11TH ASIAN CONTROL CONFERENCE (ASCC). IEEE, 17 December 2017, 1246-1251